(19) Europäisches Patentamt
European Patent Office
Office européen des brevets

(11) Veröffentlichungsnummer: **0 319 598 B1**

(12) **EUROPÄISCHE PATENTSCHRIFT**

(45) Veröffentlichungstag der Patentschrift: **25.09.91**

(51) Int. Cl.5: **A61K 31/505**, A61K 47/00

(21) Anmeldenummer: **87118082.4**

(22) Anmeldetag: **07.12.87**

# (54) Stabilisierte wässrige Folsäurezubereitung.

(43) Veröffentlichungstag der Anmeldung:
**14.06.89 Patentblatt 89/24**

(45) Bekanntmachung des Hinweises auf die Patenterteilung:
**25.09.91 Patentblatt 91/39**

(84) Benannte Vertragsstaaten:
**DE FR GB IT**

(56) Entgegenhaltungen:
**GB-A- 725 683**

(73) Patentinhaber: **Blum, Holger**
**Parkallee 75**
**W-2000 Hamburg 13(DE)**

(72) Erfinder: **Blum, Holger**
**Parkallee 75**
**W-2000 Hamburg 13(DE)**

(74) Vertreter: **Patentanwälte Kirschner & Grosse**
**Forstenrieder Allee 59**
**W-8000 München 71(DE)**

EP 0 319 598 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft eine stabilisierte wäßrige Folsäurezubereitung, die Folsäure (auch als Pteroylglutaminsäure bezeichnet) und/oder mindestens ein Ammonium-, Tetraalkylammonium-, Alkalimetall-, Erdalkalimetall- und/oder Alkanolammoniumsalz derselben enthält, mit einer verbesserten Gehaltsstabilität in Gegenwart von Sauerstoff.

Wäßrige Lösungen von Folsäure oder Salzen derselben weisen in Gegenwart von Sauerstoff nur eine begrenzte Stabilität auf. Auch in Gegenwart von Pufferlösungen wird das Folsäuremolekül innerhalb eines mehr oder weniger kurzen Zeitraums in biologisch inaktive Bruchstücke gespalten [vgl. B. Koft, G. Sevag in J. Am. Chem. Soc., 71, 3 245 (1949)]. Für die Einleitung der Zersetzung der Folsäure ist bereits eine hohe Luftfeuchtigkeit ausreichend [vgl. F.Y. Triptet, U.W. Kesselring in Pharm. Acta Helv. 1975, 50 (10), 318-322].

Aus der GB-PS 725 683 ist es bekannt, daß wäßrige Alkalimetallsalzlösungen von Folsäure durch Zugabe von Alkalimetallsalzen der Ethylendiamintetraessigsäure stabilisiert werden können. In der Praxis hat sich jedoch gezeigt, daß durch Zugabe der Alkalimetallsalze der Ethylendiamintetraessigsäure nur eine geringe stabilisierende Wirkung in Gegenwart von Luftsauerstoff erzielt werden kann. Darüber hinaus ist die Verwendung der Ethylendiamintetraessigsäure physiologisch problematisch, wenn die Vitaminzubereitung für die Tierernährung oder verwandte Einsatzgebiete verwendet werden soll.

Dort, wo wäßrige Folsäurelösungen über längere Zeiträume hinweg, beispielsweise einige Wochen lang, in Gegenwart von Luftsauerstoff gelagert werden müssen, wie dies beispielsweise bei der Behandlung von biologischen Kläranlagen der Fall ist, war man bei Anwendung der bishierigen Stabilisierungsmethoden gezwungen, einen deutlichen Abfall der Bioaktivität der wäßrigen Folsäurelösungen in Kauf zu nehmen.

Aufgabe der Erfindung war es daher, einen Weg zu finden, um wäßrige Lösungen von Folsäure und/oder Salzen derselben in Gegenwart von Sauerstoff lagerbeständig zu machen, d.h. ihnen eine länger anhaltende Stabilität zu verleihen.

Es wurde nun gefunden, daß diese Aufgabe erfindungsgemäß dadurch gelöst werden kann, daß man einer wäßrigen Folsäurezubereitung, die Folsäure und/oder ein Ammonium-, Tetraalkylammonium-, Alkalimetall-, Erdalkalimetall- und/oder Alkanolammoniumsalz derselben enthält, gleichzeitig Dihydrofolsäure und/oder ein Ammonium-, Tetraalkylammonium-, Alkalimetall-, Erdalkalimetall- und/oder Alkanolammoniumsalz derselben sowie mindestens eine Hydroxypolycarbonsäure und/oder ein Ammonium-, Tetraalkylammonium-, Alkalimetall-, Erdalkalimetall- und/oder Alkanolammoniumsalz derselben als Stabilisatoren zusetzt.

Gegenstand der Erfindung ist eine wäßrige Folsäurezubereitung, die Folsäure und/oder mindestens ein Ammonium-, Tetraalkylammonium-, Alkalimetall-, Erdalkalimetall- und/oder Alkanolammoniumsalz derselben enthält, mit verbesserter Gehaltsstabilität in Gegenwart von Sauerstoff, die dadurch gekennzeichnet ist, daß sie als Stabilisator enthält eine Kombination aus

(a) Dihydrofolsäure und/oder mindestens einem Ammonium-, Tetraalkylammonium-, Alkalimetall-, Erdalkalimetall- und/oder Alkanolammoniumsalz derselben und

(b) mindestens einer Hydroxypolycarbonsäure und/oder mindestens einem Ammonium-, Tetraalkylammonium-, Alkalimetall-, Erdalkalimetall- und/oder Alkanolammoniumsalz derselben.

Die erfindungsgemäße wäßrige Folsäurezubereitung, insbesondere wäßrige Folsäurelösung, kann über längere Zeiträume hinweg, beispielsweise einige Wochen lang, in Gegenwart von Sauerstoff gelagert werden, ohne daß ein wesentlicher Abfall ihrer Bioaktivität auftritt, d.h. sie weist eine hervorragende Lagerbeständigkeit auf, die auf die synergistische Wirkung der erfindungsgemäß verwendeten Stabilisatorkombination zurückzuführen ist und mit den bisher bekannten Stabilisatoren nicht erreichbar war.

Gemäß einer bevorzugten Ausgestaltung der Erfindung enthält die wäßrige Folsäurezubereitung die Dihydrofolsäure und/oder mindestens ein Ammonium-, Tetraalkylammonium-, Alkalimetall-, Erdalkalimetall- und/oder Alkanolammoniumsalz derselben in einer Menge von 0,001 bis 0,1 Mol, vorzugsweise in einer Menge von 0,01 bis 0,1 Mol, und die Hydroxypolycarbonsäure in einer Menge von 0,05 bis 2,5 Mol pro Mol der zu stabilisierenden Folsäure und/oder Folsäuresalze.

Bei der erfindungsgemäß verwendbaren Hydroxypolycarbonsäure handelt es sich vorzugsweise um Citronensäure, Weinsäure oder Apfelsäure.

Bei dem Alkanolammoniumsalz handelt es sich vorzugsweise um ein Salz der obengenannten organischen Säuren mit einem Dialkanolamin der Formel

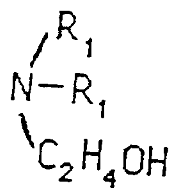

worin die Reste $R_1$ ein Wasserstoffatom und/oder Hydroxyethyl und/oder Hydroxypropyl bedeuten.

Bei der erfindungsgemäß zu stabilisierenden Folsäure, auch als Pteroylglutaminsäure bezeichnet, handelt es sich um ein Vitamin der B-Gruppe. Sie hat die Formel

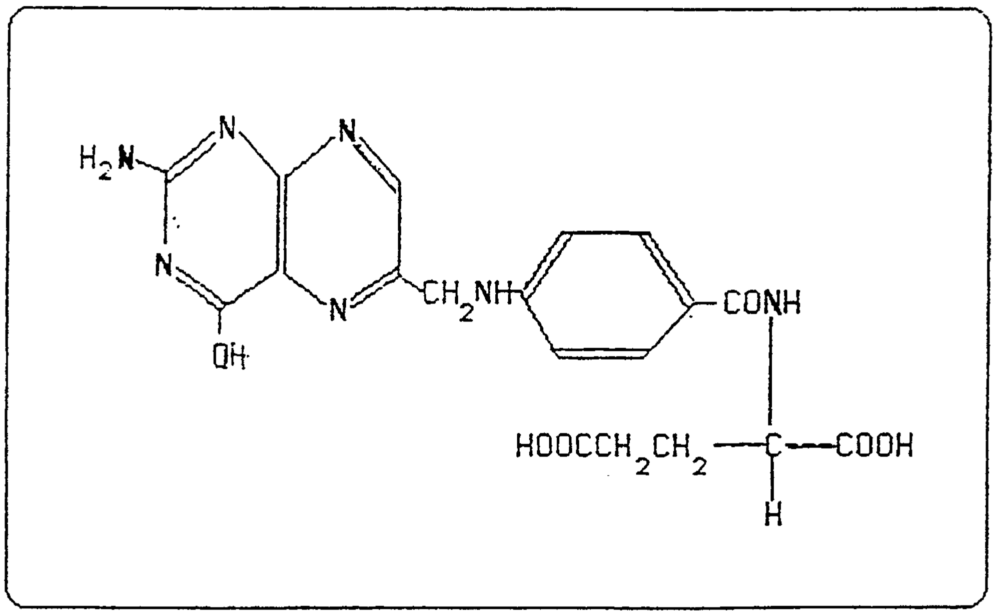

Unter den obengenannten Alkalimetallsalzen sind die Lithium-, Natrium-, Kalium-, Rubidium- und Caesiumsalze zu verstehen.

Unter dem obengenannten Ausdruck Tetraalkylammoniumsalze sind solche mit dem Kation $NR_4^{\oplus}$ zu verstehen, worin R einen niederen Alkylrest mit 1 bis 6, insbesondere 1 bis 4, speziell 1 bis 3 Kohlenstoffatomen, bedeutet.

Unter dem hier verwendeten Ausdruck "Erdalkalimetallsalze" sind die Magnesium-, Calcium-, Strontium- und Bariumsalze zu verstehen.

Bei der erfindungsgemäß zu verwenden den Dihydrofolsäure handelt es sich um eine oxidationsempfindliche Substanz, die in Gegenwart von Sauerstoff in wäßriger Lösung in Aminobenzoylglutaminsäure und Pterinsäure zerfällt. Es war daher um so überraschender, als festgestellt wurde, daß diese oxidationsempfindliche Substanz den oxidativen Zerfall der Folsäure in wäßriger Lösung zu hemmen vermag.

Die Herstellung der Dihydrofolsäure mit der nachstehend angegebenen Formel

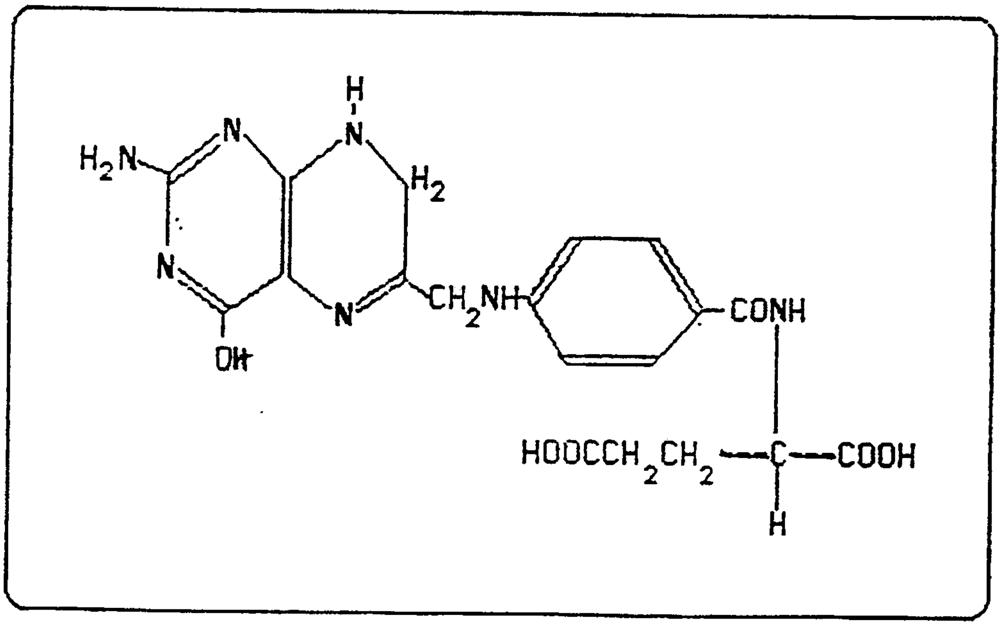

erfolgt auf an sich bekannte Weise durch partielle Reduktion von Folsäure, beispielsweise nach S. Futtermann, J. Biol. Chem., 228, 1957, Seiten 1031 ff.

Ein besonderer Vorteil der erfindungsgemäßen wäßrigen Zubereitungen besteht darin, daß die als Stabilisator verwendete Dihydrofolsäure im Gegensatz zu den bisher verwendeten bekannten Stabilisatoren für wäßrige Folsäurelösungen kein zellfremdes Material ist, sondern durch eine Reihe von Enzymen aus Folsäure als Vorstufe zu beispielsweise dem Wachstumsfaktor Citrovorum gebildet wird (vgl. E. Beilstein, III/IV, Seite 3 934).

Zur Erzielung einer ausreichenden Lagerbeständigkeit der wäßrigen Folsäurelösungen wird die zu stabilisierende Folsäure im allgemeinen mit 0,001 bis 0,1 Mol, vorzugsweise 0,01 bis 0,1 Mol Dihydrofolsäure pro Mol Folsäure versetzt. Neben der Dihydrofolsäure wird noch ein zweiter Stabilisator in Form einer Hydroxypolycarbonsäure in der waßrigen Folsäurezubereitung eingesetzt. Beispiele für geeignete Hydroxypolycarbonsäuren sind Weinsäure, Citronensäure und Apfelsäure.

Die Wirksamkeit der verschiedenen Stabilisatoren für die Lagerung wäßriger Folsäurelösungen wird bestimmt, indem man zu Beginn und am Ende der Lagerzeit Proben entnimmt und ihren Gehalt an Folsäure mikrobiologisch bestimmt. Ist die entnommene Probemenge Vitaminzubereitung in beiden Fällen gleich groß, so kann die Stabilität einfach durch Berechnung des Quotienten ermittelt werden:

$$\text{STABILITÄT} = 100 \times \frac{\text{(Folsäuregehalt am Ende der Lagerzeit)}}{\text{(Folsäuregehalt zu Beginn der Lagerzeit)}} \quad (1)$$

Verfahren zur mikrobiologischen Gehaltsbestimmung sind dem Fachmann an sich bekannt. Eine geeignete Vorschrift ist zu finden in "Official Methods of Analyses of the Association of Official Analytical Chemists", Washington, DC 20044, 13. Auflage 1980, AOAC Methods (1980), 759-763.

Die Erfindung wird durch die folgenden Beispiele näher erläutert.

Beispiel 1

Zur Verdeutlichung der stabilisierenden Wirkung von Dihydrofolsäure in synergistischer Kombination mit Hydroxypolycarbonsäuren wurden verschiedene wäßrige Folsäurezubereitungen mit den nachstehend angegebenen Zusammensetzungen hergestellt.

Jeweils 100 g der so hergestellten Folsäurezubereitungen wurden über den in der nachstehenden Tabelle I angegebenen Zeitraum in einer offenen braunen Glasflasche mit einem Nettoinhalt von 280 ml bei Raumtemperatur aufbewahrt. Am Ende der Lagerperiode wurde der gemessene Verdunstungsverlust mit

Wasser wieder aufgefüllt und es wurde der Gehalt an Folsäure gemäß den AOAC Methods mikrobiologisch bestimmt. Die Berechnung der Stabilität erfolgte nach der vorstehend angegebenen Formel (1).

Zubereitung A

Es wurde eine wäßrige Lösung von Folsäure, Dihydrofolsäure und Citronensäure unter Verwendung von Natronlauge hergestellt. Der pH-Wert der Lösung betrug 10,4. 100 g Lösung enthielten 16 mMol Folsäuredinatriumsalz, 1 mMol Dihydrofolsäurenatriumsalz und 5 mMol Trinatriumcitrat.

Zubereitung B

Es wurde eine wäßrige Lösung von Folsäure und Dihydrofolsäure unter Verwendung von Natronlauge hergestellt. Der pH-Wert der Lösung betrug 10,2. 100 g Lösung enthielten 16 mMol Folsäuredinatriumsalz und 4 mMol Dihydrofolsäurenatriumsalz.

Zubereitung C

Es wurde eine wäßrige Lösung von Folsäure, Dihydrofolsäure und Citronensäure unter Verwendung von Natronlauge hergestellt. Der pH-Wert der Lösung betrug 11,2. 100 g Lösung enthielten 16 mMol Folsäuredinatriumsalz, 0,1 mMol Dihydrofolsäurenatriumsalz und 10 mMol Trinatriumcitrat.

Zubereitung D

Es wurde eine wäßrige Lösung von Folsäure und Citronensäure unter Verwendung von Natronlauge hergestellt. Der pH-Wert der Lösung betrug 10,3. 100 g Lösung enthielten 16 mMol Folsäuredinatriumsalz und 12 mMol Trinatriumcitrat.

Zubereitung E

Es wurde eine wäßrige Lösung von Folsäure, Dihydrofolsäure und Citronensäure unter Verwendung von Natronlauge und Calciumhydroxid hergestellt. Der pH-Wert der Lösung betrug 10,4. 100 g Lösung enthielten 5 mMol Folsäurecalciumsalz, 1 mMol Dihydrofolsäurenatriumsalz und 7 mMol Trinatriumcitrat.

Zubereitung F

Es wurde eine wäßrige Lösung von Folsäure, Dihydrofolsäure und Citronensäure unter Verwendung von Diethanolamin hergestellt. Der pH-Wert der Lösung betrug 9,6. 100 g Lösung enthielten 22 mMol Folsäurediethanolaminsalz, 2 mMol Dihydrofolsäurediethanolaminsalz und 8,2 mMol Citronensäurediethanolaminsalz.

Zubereitung G

Es wurde eine wäßrige Lösung von Folsäure, Dihydrofolsäure und Weinsäure unter Verwendung von Natronlauge hergestellt. Der pH-Wert der Lösung betrug 10,4. 100 g Lösung enthielten 16 mMol Folsäuredinatriumsalz, 4 mMol Dihydrofolsäurenatriumsalz und 4 mMol Weinsäuredinatriumsalz.

Zubereitung H

Es wurde eine wäßrige Lösung von Folsäure und Ethylendiamintetraessigsäure unter Verwendung von Natronlauge und Kalilauge hergestellt. Der pH-Wert der Lösung betrug 11. 100 g Lösung enthielten 16 mMol Folsäuredinatriumsalz und 8 mMol Ethylendiamintetraessigsäuretetrakaliumsalz.

Zubereitung I

Es wurde eine wäßrige Lösung von Folsäure und Ethylendiamintetraessigsäure unter Verwendung von Natronlauge und Kalilauge hergestellt. Der pH-Wert der Lösung betrug 11. 100 g Lösung enthielten 16 mMol Folsäuredinatriumsalz und 16 mMol Ethylendiamintetraessigsäuretetrakaliumsalz.

Zubereitung J

Es wurde eine wäßrige Lösung von Folsäure, Dihydrofolsäure und Citronensäure unter Verwendung von Kalilauge und Calciumhydroxid hergestellt. Der pH-Wert der Lösung betrug 10,8. 100 g Lösung enthielten 5 mMol Folsäurecalciumsalz, 0,2 mMol Dihydrofolsäurekaliumsalz und 3 mMol Trikaliumcitrat.

Zubereitung K

Es wurde eine wäßrige Lösung von Folsäure, Dihydrofolsäure und Weinsäure sowie Ethylenglykol unter Verwendung von Natronlauge hergestellt. Der pH-Wert der Lösung betrug 10,4. 100 g Lösung enthielten 16 mMol Folsäuredinatriumsalz, 1 mMol Dihydrofolsäurenatriumsalz, 4 mMol Weinsäuredinatriumsalz sowie 300 mMol Ethylenglykol.

Zubereitung L

Es wurde eine wäßrige Lösung von Folsäure, Dihydrofolsäure und Citronensäure unter Verwendung von Natronlauge und Kalilauge hergestellt. Der pH-Wert der Lösung betrug 10,4. 100 g Lösung enthielten 8 mMol Folsäuredinatriumsalz, 8 mMol Dihydrofolsäurenatriumsalz und 5 mMol Trikaliumcitrat.

Zubereitung M

Es wurde eine wäßrige Lösung von Folsäure, Dihydrofolsäure und Ethylendiamintetraessigsäure unter Verwendung von Natronlauge hergestellt. Der pH-Wert der Lösung betrug 10,4. 100 g Lösung enthielten 10 mMol Folsäuredinatriumsalz, 1 mMol Dihydrofolsäurenatriumsalz und 6 mMol Ethylendiamintetraessigsäuretetranatriumsalz.

Zubereitung N

Es wurde eine wäßrige Lösung von Folsäure, Dihydrofolsäure und Ethylendiamintetraessigsäure unter Verwendung von Natronlauge hergestellt. Der pH-Wert der Lösung betrug 10,4. 100 g Lösung enthielten 10 mMol Folsäuredinatriumsalz, 4 mMol Dihydrofolsäurenatriumsalz und 8 mMol Ethylendiamintetraessigsäuretetranatriumsalz.

Zubereitung O

Es wurde eine wäßrige Lösung von Folsäure, Dihydrofolsäure und Glykolsäure unter Verwendung von Natronlauge hergestellt. Der pH-Wert der Lösung betrug 10,4. 100 g Lösung enthielten 16 mMol Folsäuredinatriumsalz, 1 mMol Dihydrofolsäurenatriumsalz und 10 mMol Natriumglykolat.

Zubereitung P

Es wurde eine wäßrige Lösung von Folsäure, Dihydrofolsäure und Bernsteinsäure unter Verwendung von Natronlauge hergestellt. Der pH-Wert der Lösung betrug 10,4. 100 g Lösung enthielten 16 mMol Folsäuredinatriumsalz, 1 mMol Dihydrofolsäurenatriumsalz und 10 mMol Dinatriumsuccinat.

Zubereitung Q

Es wurde eine wäßrige Lösung von Folsäure, Dihydrofolsäure und Apfelsäure unter Verwendung von Natronlauge hergestellt. Der pH-Wert der Lösung betrug 10,4. 100 g Lösung enthielten 16 mMol Folsäuredinatriumsalz, 1 mMol Dihydrofolsäurenatriumsalz und 10 mMol Apfelsäuredinatriumsalz.

Zubereitung R

Es wurde eine wäßrige Lösung von Folsäure unter Verwendung von Natronlauge hergestellt. Der pH-Wert der Lösung betrug 10,4. 100 g Lösung enthielten 16 mMol Folsäuredinatriumsalz.

Wie aus der nachstehenden Tabelle I ersichtlich, wiesen die erfindungsgemäßen Zubereitungen A, C, E, F, G, J, K,
L und Q eine sehr gute Lagerbeständigkeit auf im Gegensatz zu den anderen folsäurehaltigen Zubereitungen, die keinen oder einen nicht-erfindungsgemäßen Stabilisator enthielten.

Tabelle I

| Zubereitung | Lagerzeit (Tage) | Stabilität (%) | Bemerkungen |
|---|---|---|---|
| A | 280 | 92 | erfindungsgemäß |
| B | 280 | 78 | |
| C | 280 | 95 | erfindungsgemäß |
| D | 280 | 75 | |
| E | 280 | 91 | erfindungsgemäß |
| F | 200 | 92 | erfindungsgemäß |
| G | 280 | 88 | erfindungsgemäß |
| H | 280 | 62 | |
| I | 280 | 65 | |
| J | 260 | 87 | erfindungsgemäß |
| K | 280 | 89 | erfindungsgemäß |
| L | 260 | 96 | erfindungsgemäß |
| M | 200 | 75 | |
| N | 200 | 76 | |
| O | 200 | 72 | |
| P | 200 | 68 | |
| Q | 200 | 82 | erfindungsgemäß |
| R | 200 | 30 | |

Beispiel 2

Jeweils 100 g der Folsäurezubereitungen A und R mit den in den obigen Beispielen angegebenen Zusammensetzungen wurden 280 Tage lang in einer offenen braunen Glasflasche mit einem Nettoinhalt von 280 ml bei Raumtemperatur aufbewahrt. Am Ende der Lagerperiode wurde der gemessene Verdunstungsverlust durch Wasser wieder ausgeglichen.

Die Folsäurezubereitung wurde dann einer stark belasteten Belebtschlamm/Abwasser-Probe (BTS = 0,8), entnommen aus einem kommunalen Klärwerk, im Verhältnis 0,26 mg Folsäurezubereitung pro Liter Schlamm/Wasser-Suspension zugesetzt. Die Suspension wurde mit 150 mg/l Phenol vergiftet und die Atmungsaktivität des vergifteten Belebtschlammes wurde in einer Warburg-Apparatur gemessen. Das Ergebnis ist in der nachstehenden Tabelle II angegeben.

Tabelle II

| Folsäurezubereitung | $Y^*_{max}$-Wert (mg $O_2$/gN, min) | Veränderung gegenüber Blindversuch (%) |
|---|---|---|
| keine | 10,4 | - |
| "A" | 18,4 | +77 |
| "R" | 11,6 | +12 |

Die die erfindungsgemäß stabilisierte Zubereitung A enthaltende Belebtschlammprobe wies gegenüber der die nichtstabilisierte Zubereitung R enthaltenden Belebtschlammprobe eine wesentlich größere Abbaurate auf.

Beispiel 3

Es wurde eine Folsäurezubereitung hergestellt durch inniges Mischen von 470 g Folsäuredihydratcalciumsalz, 30 g Dihydrofolsäurehydratcalciumsalz und 500 g Citronensäuredihydrat. 340 g feuchtes Blutmehl, enthaltend 200 g Wasser und 10 g der vorstehend angegebenen Folsäurezubereitung wurden bei 80 °C miteinander gemischt und auf der Walze getrocknet.

Das erhaltene Produkt wies eine Restfeuchte von 13 % und einen Folsäuregehalt, mikrobiologisch bestimmt, von 2,40 Gew.-% auf. Nach 6-monatiger Lagerung bei 25° C betrug der mikrobiologisch bestimmte Folsäuregehalt noch 2,15 Gew.-%, entsprechend 90 % des ursprünglichen Wertes.

Beispiel 4 (Vergleichsbeispiel)

340 g feuchtes Blutmehl, enthaltend 200 g Wasser, und 5 g Folsäuredihydrat wurden bei 80° C miteinander gemischt und auf einer Walze getrocknet.

Das erhaltene Produkt wies eine Restfeuchte von 14 % und einen Folsäuregehalt, mikrobiologisch bestimmt, von 3,0 Gew.-% auf. Nach 6-monatiger Lagerung bei 25° C betrug der mikrobiologisch bestimmte Folsäuregehalt noch 1,9 Gew.-%, entsprechend 63 % des ursprünglichen Wertes.

## Patentansprüche

1. Wäßrige Folsäurezubereitung, die Folsäure und/oder mindestens ein Ammonium-, Tetraalkylammonium-, Alkalimetall-, Erdalkalimetall- und/oder Alkanolammoniumsalz derselben enthält, mit verbesserter Gehaltsstabilität in Gegenwart von Sauerstoff, dadurch gekennzeichnet, daß sie als Stabilisator enthält eine Kombination aus

   (a) Dihydrofolsäure und/oder mindestens einem Ammonium-, Tetraalkylammonium-, Alkalimetall-, Erdalkalimetall- und/oder Alkanolammoniumsalz derselben und

   (b) mindestens einer Hydroxypolycarbonsäure und/oder mindestens einem Ammonium-, Tetraalkylammonium-, Alkalimetall-, Erdalkalimetall- und/oder Alkanolammoniumsalz derselben.

2. Folsäurezubereitung nach Anspruch 1, dadurch gekennzeichnet, daß sie die Dihydrofolsäure und/oder mindestens ein Ammonium-, Tetraalkylammonium-, Alkalimetall-, Erdalkalimetall- und/oder Alkanolammoniumsalz derselben in einer Menge von 0,001 bis 0,1 Mol, vorzugsweise von 0,01 bis 0,1 Mol, und die Hydroxypolycarbonsäure und/oder mindestens ein Ammonium-, Tetraalkylammonium-, Alkalimetall-, Erdalkalimetall- und/oder Alkanolammoniumsalze derselben in einer Menge von 0,05 bis 2,5 Mol, jeweils pro Mol Folsäure und/oder Ammonium-, Tetraalkylammonium-, Alkalimetall-, Erdalkalimetall- und/oder Alkanolammoniumsalz derselben, enthält.

3. Folsäurezubereitung nach Anspruch 1 oder 2, dadurch gekennzeichnet, daß sie als Hydroxypolycarbonsäure Citronensäure, Weinsäure und/oder Apfelsäure enthält.

4. Folsäurezubereitung nach einem der Ansprüche 1 bis 3, dadurch gekennzeichnet, daß es sich bei dem Alkanolammoniumsalz um ein Salz der organischen Säure mit einem Alkanolamin der Formel handelt

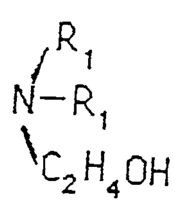

worin die Reste $R_1$ ein Wasserstoffatom und/oder Hydroxyethyl und/oder Hydroxypropyl bedeuten.

## Claims

1. Aqueous folic acid preparation containing folic acid and/or at least one ammonium salt, tetraalkylammonium salt, alkali metal salt, alkaline earth metal salt and/or alkanol ammonium salt thereof and having an improved content stability in the presence of oxygen, **characterized in that** it contains as stabilizer a combination of

   (a) dihydrofolic acid and/or at least one ammonium salt, tetraalkylammonium salt, alkali metal salt, alkaline earth metal salt and/or alkanol ammonium salt thereof and

   (b) at least one hydroxypolycarboxylic acid and/or at least one ammonium salt, tetraalkylammonium salt, alkali metal salt, alkaline earth metal salt and/or alkanol ammonium salt thereof.

2. Folic acid preparation as defined in claim 1, **characterized in that** it contains said dihydrofolic acid and/or at least one ammonium salt, tetraalkylammonium salt, alkali metal salt, alkaline earth metal salt and/or alkanol ammonium salt thereof in an amount of 0.001 to 0.1 mol, preferably of 0.01 to 0.1 mol, and said hydroxypolycarboxylic acid and/or at least one ammonium salt, tetraalkylammonium salt, alkali metal salt, alkaline earth metal salt and/or alkanol ammonium salt thereof in an amount of 0.05 to 2.5 mol, respectively, per mol of folic acid and/or ammonium salt, tetraalkylammonium salt, alkali metal salt, alkaline earth metal salt and/or alkanol ammonium salt thereof.

3. Folic acid preparation as defined in claim 1 or 2, **characterized in that** it contains citric acid, tartaric acid and/or malic acid as hydroxypolycarboxylic acid.

4. Folic acid preparation as defined in one of claims 1 to 3, **characterized in that** the alkanol ammonium salt is a salt of the organic acid with an alkanol amine of the formula

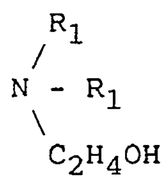

wherein the radicals $R_1$ represent a hydrogen atom and/or hydroxyethyl and/or hydroxypropyl.

## Revendications

1. Préparation aqueuse d'acide folique, qui contient l'acide folique et/ou au moins un sel d'ammoniac, de tétraalkylammoniac, un sel des métaux alcalins, un sel des métaux alcalino-terreux et/ou un sel d'ammoniac d'alcanol, présentant une stabilité améliorée en présence d'oxygène, caractérisée en ce qu'elle contient en tant que stabilisateur une combinaison de
(a) acide dihydrofolique et/ou au moins un sel d'ammoniac, de tétraalkylammoniac, des métaux alcalins, des métaux alcalino-terreux et/ou un sel d'ammoniac d'alcanol de ceux-ci et
(b) au moins un acide hydroxypolycarboxylique et/ou au moins un sel d'ammoniac, de tétraalkylammoniac, de métaux alcalins, de métaux alcalino-terreux et/ou leur sel d'ammoniac d'alcanol.

2. Préparation d'acide folique selon la revendication 1, caractérisée en ce qu'elle comprend l'acide dihydrofolique et/ou au moins un sel d'ammoniac, de tétraalkylammoniac, de métaux alcalins, de métaux alcalino-terreux et/ou leur sel d'ammoniac d'alcanol dans une quantité de 0,001 jusqu'à 0,1 mol, de préférence de 0,01 jusqu'à 0,1 mol, et l'acide hydroxypolycarboxylique et/ou au moins un sel d'ammoniac, de tétraalkylammoniac, de métaux alcalins, de métaux alcalino-terreux et/ou leurs sels d'ammoniac d'alcanol dans une quantité de 0,05 jusqu'à 2,5 mol, respectivement par mol l'acide folique et/ou le sel d'ammoniac, le tétraalkylammoniac, les métaux alcalins, les métaux alcalino-terreux et/ou leur sel d'ammoniac d'alcanol.

3. Préparation d'acide folique selon la revendication 1 ou 2, caractérisée en ce qu'en tant qu'acide hydroxypolycarboxylique elle contient l'acide citrique, l'acide tartrique et/ou l'acide maléïque.

4. Préparation d'acide folique selon l'une des revendications 1 à 3, caractérisée en ce que pour le sel d'ammoniac d'alcanol il s'agit d'un sel de l'acide organique avec une alcanolamine de la formule

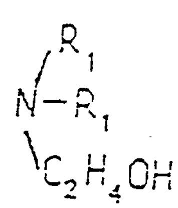

dans laquelle les radicaux $R_1$ représentent un atome d'oxygène et/ou l'hydroxyéthyle et/ou l'hydroxypropyle.